# EUROPEAN PATENT APPLICATION

(11) **EP 4 205 642 A1**
(43) Date of publication of application: **05.07.2023**
(21) Application number: 21218038.4
(22) Date of filing: 29.12.2021
(51) Int. Cl.: A61B 5/01, A61B 5/026

(54) **BLOOD FLOW RATE ESTIMATION USING CFD SIMULATIONS**

(71) Applicant: Koninklijke Philips N.V., 5656 AG Eindhoven (NL)
(72) Inventor: FRACKOWIAK, Bruno Jean François, Eindhoven (NL); VAN DER HORST, Arjen, Eindhoven (NL); STEINBUCH, Jeire, Eindhoven (NL)
(74) Representative: Philips Intellectual Property & Standards

(57) **Abstract**

A system is provided for estimating the blood flow rate in the vascular system of a subject, the system comprising a processor configured to execute memorized instructions to obtain at least two temperature measurements over time of at least two positions in the vascular system and generate a plurality of computational fluid dynamic, CFD, simulations of blood flow and temperatures in the vascular system using the geometry of the vascular system as boundary conditions, wherein each CFD simulation corresponds to respective CFD input parameters. The processor is further configured to obtain, from each CFD simulation, simulated temperatures over time at positions corresponding to the positions in the vascular system and estimate the blood flow rate in the vascular system including comparing the temperature measurements over time to the simulated temperatures over time corresponding to different CFD input parameters.

## Description

### FIELD OF THE INVENTION

The invention relates to the field of estimating or measuring the blood flow rate in the vascular system of subjects.

### BACKGROUND OF THE INVENTION

The blood flow in humans ensures the transportation of nutrients, hormones, metabolic waste products, oxygen, and carbon dioxide throughout the body to maintain cell-level metabolism, the regulation of the pH, osmotic pressure and temperature of the whole body, and the protection from microbial and mechanical harm.

The heart is the driver of the circulatory system, pumping blood through rhythmic contraction and relaxation. The rate of blood flow out of the heart is known as the cardiac output.

Blood being pumped out of the heart first enters the aorta, the largest artery of the body. It then proceeds to divide into smaller and smaller arteries, then into arterioles, and eventually capillaries, where oxygen transfer occurs. Thus, the cardiac output from the heart is divided into the smaller arteries. The particular blood flow rate through an artery will typically depend on the diameter of the artery and the cardiac output.

Blood flow rate can be measured through imaging techniques or using intravascular devices. However, obtaining accurate blood flow measurements from these methods is typically a challenging task. Blood flow measurements using these methods can be affected by patient motion artefacts, limited time resolution, sensor orientation, signal noise, complexity of the device, user variability, distorted vessel tree and the pulsatile nature of the blood flow.

Despite the difficulty in obtaining accurate blood flow rate measurements, the clinical information which can be derived from blood flow rate measurements can be very valuable for a clinician. For example, blood flow rates can be used to assess vascular diseases (e.g. in peripheral artery disease (PAD) or coronary artery disease (CAD)).

Thus, there is a need for improved methods for measuring the blood flow rate.

It is for instance known, among other techniques, to embed in an endovascular device, an optical fiber provided with temperature-sensitive optical sensor segments spatially distributed along its length, to sense changes of temperature, for instance induced by the injection of a bolus, and thus changes of blood flow rates along its length.

Now, since the temperature naturally and significantly changes over the width of a vessel (wall effects), and the bolus is more difficult to detect at locations far from the injection point (thermal diffusion and flow mixing effects), there is a need to increase the reliability of temperature and blood flow rate measurement.

### SUMMARY OF THE INVENTION

The invention is defined by the claims.

According to examples in accordance with an aspect of the invention, there is provided a system for estimating the blood flow rate in the vascular system of a subject, the system comprising a processor configured to execute memorized instructions to:
receive at least two temperature measurements over time of at least two positions in the vascular system;
generate a plurality of computational fluid dynamic, CFD, simulations of temperatures in the vascular system using the geometry of the vascular system as boundary conditions, wherein each CFD simulation corresponds to respective CFD input parameters which are associated with blood flow features (e.g. pressure and/or resistance and/or impedance as exemplary described in below specification, and/or temperature in case a bolus injection is further modelled (or simulated) - specific embodiment further explained below);
obtain, from each CFD simulation, simulated temperatures over time at positions corresponding to the positions in the vascular system; and
estimate the blood flow rate in the vascular system by comparing the temperature measurements over time to the simulated temperatures over time associated with different CFD input parameters.

In order to estimate the real blood flow rate, CFD simulations are performed for different CFD input parameters. Different CFD input parameters will alter the simulated temperatures. Thus, the simulated temperatures which are most similar to the actual temperature measurements should indicate that the CFD input parameters which correspond to said, most similar, simulated temperatures may be the closest to the real values. The CFD input parameters are associated with blood flow features (such as flow rate or pressure), so by determining the blood flow features which result in the best CFD simulation, the actual blood flow rate can be estimated.

The CFD input parameters may comprise a blood flow rate to be simulated (i.e. a blood flow rate to be modeled). In this case, once the best matching CFD simulation is identified, the CFD input parameters directly provide the estimated blood flow rate. However, the CFD parameters may instead provide other blood flow features. In such a case, once the best matching CFD simulation has been identified, the simulation itself may yield the estimated blood flow rate as another output of the CFD simulation (i.e. in addition to the simulated temperatures).

In all cases, the CFD input parameters alter blood flow characteristics being simulated. Thus, the simulated temperatures are also altered as any changes in temperature will also change relative to the blood flow. By finding the "closest" CFD input parameters, the best simulation is found so that the CFD input parameters may be assumed to apply to the real blood flow.

Additionally, the CFD simulations may be used to provide accurate simulations of blood flow in the vascular system. The flow of blood in a vessel is not trivial and thus direct blood flow measurements can be difficult or even erroneous in some cases. For example, the velocity of the blood flow can vary depending on how close to the wall the measurement is taken.

The system may further comprise a temperature measurement system for measuring the at least two temperature measurements.

The positions at which the at least two temperature measurements are taken may be known from the system architecture, estimated or measured. The system may for example comprise a localization system for obtaining the positions. For example, a fiber optic real shape (FORS) device may be used as the temperature measurement system and localization system. This is based on force measurements, but other ways to track position may be used.

It has been realized by the inventors that one way of comparing the CFD simulations to the real blood flow is by comparing the temperature changes along the length of a FORS device when a bolus e.g. a cold bolus is injected. The bolus is a single dose of a drug or other substance given over a short period of time (e.g. by injection into the blood vessel). A cold bolus is a bolus at a temperature lower than the temperature of the blood (i.e. lower than 37°C). Preferably, the cold bolus is at a temperature lower than 30°C or 25°C. The lower the temperature of the cold bolus, the higher the contrast in temperature between the cold bolus and blood (up to a limit). The cold bolus will join the blood flow and gradually warm up to the temperature of the blood.

The temperature measurements may thus correspond to changes in temperature in the vascular system caused by the injection of a cold bolus, obtained by temperature sensing embedded in an interventional device.

The interventional device may further comprise a tracking device provided with temperature sensing to obtain said temperature measurements, and arranged to measure the positions of said at least two temperature measurements. For example, ultrasound transducers, electromagnetic (EM) sensors, optical sensors (e.g. Bragg gratings, Rayleigh scattering elements, provided in an optical fiber used in said device) may be provided with a known position with respect to the temperature sensors, with an external ultrasound probe, EM detectors, optical interrogators, respectively, to capture positions based on detected ultrasound /EM/optical data. Alternatively the position may be obtained by an imaging process based on optical markers provided on the interventional device.

As a particular embodiment, the temperature measurements may be obtained from shape sensors provided in the interventional device, such that shape is determined together with said positions (the latter may be obtained by the same shape sensors if they are registered to a known coordinates).

Shape sensing may be provided in a fiber optic real shape (FORS) device. The positions may be obtained by obtaining a geometry of the FORS device inside the vascular system or shape information registered to an external coordinate system in which the positions are determined. FORS devices are provided in thin, elongated and flexible interventional devices (i.e. similar to catheters or guidewires). FORS devices are manufactured such that the shape and geometry of the FORS device can be obtained from received optical data, even when the FORS device is not visible in the image obtained from the imaging system and without the FORS device having to be imaged. A FORS device comprises a plurality of optical fibers or one optical fiber provided with a plurality of optical cores, provided with a plurality of optical sensors (e.g. Bragg gratings or Rayleigh scatter sensing elements), and the cores are arranged typically wounded about a central axis, core or fiber, to reconstruct a shape, running through at least a part of the length of the FORS device, in well-known manner.

At any particular geometry of the FORS device, changes of light reflected from or scattered within the optical fibers are associated with internal strains, thus enabling the geometry of the FORS device to be reconstructed.

FORS technology offers the advantage of tracking the full length, or a part of the length, of the catheter/guidewire over the blood vessel, in a known coordinate system if registered thereto. Hence the catheter/guidewire can be included as a bluff body in the CFD simulations, which makes them more accurate.

The FORS device may also be used to measure at least two temperatures in a known manner, at a few points or all along the length of the FORS device. The temperature sensing elements may be positioned at different positions along the FORS device to measure the temperature at different parts of the FORS device.

As the cold bolus joins the blood flow, the FORS device will detect lower temperatures along the length of the FORS device at different times (e.g. as the cold bolus travels down the length of the FORS device). The different temperatures along the length of the FORS device can thus indicate the blood flow rate.

Thus, the temperature measurements of the FORS device corresponding to the injection of the cold bolus can be compared to the simulated temperatures and, the most similar simulated temperatures can be used to indicate an estimate of the real blood flow rate (based on the corresponding CFD input parameters). Each simulated temperature is obtained from a different CFD simulation and each CFD simulation is based on CFD input parameters which correspond to a different blood flow conditions such as blood flow rate. Thus, the most similar simulated temperatures correspond to the simulated blood flow rates which are most likely to be correct. The blood flow rate may comprise an average blood flow rate over time.

The FORS device is capable of outputting the geometry of the FORS device and at least two temperature measurements along the length of the FORS device.

The processor for example obtains, from the FORS device, a geometry of the FORS device inside the vascular system and temperature measurements along the length of the FORS device over time.

The geometry of the vascular system may be obtained from image data of the vascular system (e.g. computed tomography angiography, magnetic resonance angiography, rotational angiography, intravascular ultrasound or any other intravascular imaging device). The geometry of the vascular system may also be obtained from a model of the vascular system. The geometry of the vascular system may be a computer generated 3D model of the vascular system. The model may be used as boundary conditions in the CFD simulations. The vascular model could also be generated based on FORS data associated with the geometry of the FORS device. The determined shape may also or alternatively be optionally used to identify an anatomical region of the vasculature system (certain vessels or organs have indeed well recognizable anatomical features).

Note that shape sensing is not necessarily a FORS-type shape sensing, other technologies could be used such as for instance shape determination based on sensed nodes of the interventional device (using for instance embedded ultrasound or EM sensors - as above-mentioned, using interpolation between nodes to determine the shape).

An injection volume and an injection time may also be provided corresponding to the injection of the cold bolus into the vascular system for use in the simulation of the injection of the cold bolus. For example, the injection volume and time are known even for a manual bolus injection. An injection flow rate can also be obtained, for example when using an injection pump. Thus, the simulations take into account of the injection of the cold bolus into the vascular system, in particular the volume and injection time of the cold bolus and possibly also flow characteristics. The injection position of the cold bolus may also be obtained, indicating where the cold bolus was injected in the vascular system. The precise injection parameters (e.g. injection time, injection location, flow rate of injection and/or injection volume) may not always be known and may have to be estimated or a generic parameter may be used. For example, the injection time may be estimated from temperature measurements indicating a drop in temperature. The injection position may be upstream of the positions such that the temperature changes induced by the injection can be measured.

The vessels which may be imaged may depend on the particular method of obtaining the temperature measurements and size considerations. For example, the vessels which may be imaged with a FORS device may depend on the size (e.g. diameter) of the particular FORS device. However, it is envisioned that with the appropriate methods and tools for obtaining the temperature measurements, the blood flow in most vessels in the vascular system of a subject could be estimated.

Thus, in summary, the CFD simulations are meant to mimic the flow of blood in the vascular system of the subject, injection of the cold bolus in the vascular system and subsequent temperature measurements along the length of the FORS device. The only different "parameter" between reality and the simulation is meant to be the blood flow rate.

Simulated temperatures can thus be obtained for each CFD simulation (therefore corresponding to a different estimated blood flow rate). The temperature measurements may be compared to the simulated temperatures using least square fitting methods (e.g. Levenberg-Marquardt, Gauss-Newton, Gradient methods, Direct search methods, etc.).

Different methods may be used to determine how many CFD simulations to generate. For example, a pre-determined number of CFD simulations may be performed in parallel or sequentially for a set of estimated blood flow rates.

The processor may be configured to generate the CFD simulations by iteratively generating CFD simulations with CFD input parameters which are generated based on a comparison of the simulated temperatures of the previous CFD simulations with the temperature measurements, wherein the first CFD simulation of the iterative generation of CFD simulations is generated using first CFD input parameters.

In essence, the CFD input parameters are optimized over the iterative CFD simulations.

Iterative generation of the CFD simulations enables a quick convergence of the CFD input parameters to the real values. The number of iterations may be dependent on the time it takes for a particular processing system to generate the CFD simulations. Additionally, the dependence of the estimated blood flow values on previous comparisons enables a more accurate determination of the real blood flow rate.

The CFD input parameters for the CFD simulations may comprise estimated/simulated blood flow rates as explained above. The blood flow rate simulated at the CFD simulations can thus be estimated and simulated temperature maps can be obtained for each different estimated blood flow rate. As before, the estimated blood flow rates may be iteratively optimized. The estimated blood flow rates may comprise periodic pulses based on flow rate profiles of blood from the heart.

Blood flow in the vascular system is not constant and is dependent on the period flow of blood from the heart. The flow of blood from the heart is typically formed or periodic pulses forming a particular profile. This profile can be used to inform the CFD simulations how to vary the blood flow over time and thus obtain more precise simulated temperatures. Generic velocity/flow profiles for particular vessels may be used depending on which vessel the measurements are performed.

The estimated blood flow rates may be based on measurements of the heart rate of the subject. As the heart rate of the subject may vary over time, these variations in heart rate can also be used to inform the CFD simulations the rate at which to simulate the pulses of blood and how this rate changes over time, further increasing the precision of the CFD simulations and therefore further increases the precision of the simulated temperatures.

The CFD input parameters for the CFD simulations may comprise a measurement of the pressure in the vascular system used as a boundary condition for the CFD simulations and estimated outlet resistance values.

Using the outlet resistances as the CFD input parameters may be more accurate than using estimated blood flow values as pressure measurements are also used and these provide further boundary conditions for the CFD simulations. Without a pressure measurement, the outlet resistance is determined based on population data for example.

The processor may be further configured to estimate outlet resistance values in the vascular system including comparing the temperature measurements over time to the simulated temperatures over time corresponding to different CFD input parameters.

The outlet resistance value provides valuable insight into the health of the vessel (e.g. diffuse stenosis, hypertension, microembolization etc.). The particular health of the vessel may also affect the blood flow rate through the vessel which, in turn, affects the blood flow rate throughout the whole vascular system.

The pressure measurements may comprise pressure measurements over time (i.e., time varying pressure measurements).

The inclusion of time varying pressure measurements as an additional boundary condition to the CFD simulations improves the accuracy of the outlet resistance value(s) output from the CFD simulations. Similarly to the blood flow, pressure in a vessel is not typically constant and it influenced by the regulation mechanism for example. Thus, including the time varying nature of the pressure as a boundary condition of the CFD simulation further increases the accuracy of the estimated blood flow rate and/or outlet resistance.

Comparing the temperature measurements to simulated temperatures may comprise inputting the temperature measurements and simulated temperatures into a pattern detection algorithm and comparing the detected patterns.

Pattern detection algorithms may be artificial intelligence algorithms, machine learning algorithms trained on datasets or deep learning algorithms with a neural network. The pattern detection algorithm may be trained on a population dataset (i.e. data from real measurements) and/or on a simulated dataset (i.e. data from simulated measurements). Pattern detection algorithms are typically quicker and more accurate/robust than other methods (e.g. least square method).

The system may further comprise a shape sensing device for obtaining the at least two temperature measurements and a geometry of the shape sensing device relative to the vascular system, wherein the geometry of the shape sensing device comprises the measurement positions of the corresponding temperature measurements. For example, the shape sensing device may be a FORS device.

Being able to obtain the temperature measurements and the geometry of the device removes the need for having two systems to measure the temperature and localize the measurements separately. In some cases, it may be possible for the user to input the positions into, for example, a user interface, which also removes the need for a localization system. The shape sensing can also be used to identify location such as the organ in which the system is located.

The system may further comprise an imaging system for obtaining the geometry of the vascular system.

The CFD simulations may further include a simulation of an injection of a temperature altering bolus. In that case this temperature resulting from a modelled injection may be an additional CFD input parameters of said CFD simulations, and the CFD simulation being then configured, to obtain temperatures in the vascular system.

The processor may be further configured to analyze quality parameters of the temperature measurements and output a temperature measurement feedback to a user interface.

In some cases, the temperature measurements may not be adequate for the comparison and thus would not lead to accurate estimations. The quality parameters may be the length in time of the temperature measurements, the temperature differences in the temperature measurements etc. The processor may then output the temperature measurement feedback (e.g. "measurement OK", "re-take measurement" etc.).

The invention also provides a method for estimating the blood flow rate in the vascular system of a subject, the method comprising:
obtaining at least two temperature measurements over time of at least two positions in the vascular system;
generating a plurality of computational fluid dynamic, CFD, simulations of blood flow and temperatures in the vascular system using the geometry of the vascular system as boundary conditions, wherein each CFD simulation corresponds to respective CFD input parameters which are associated with blood flow features;
obtaining, from each CFD simulation, simulated temperatures over time at positions corresponding to the positions in the vascular system; and
estimating the blood flow rate in the vascular system by comparing the temperature measurements over time to the simulated temperatures over time corresponding to different CFD input parameters.

The invention also provides a computer program product comprising computer program code which, when executed on a computing device having a processing system, cause the processing system to perform all of the steps of the afore-mentioned method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an illustration of a fiber optic real shape, FORS, device for measuring the temperature inside a vessel and corresponding simulated temperatures;
Figure 2 shows simulated temperatures and experimental results;
Figure 3 illustrates one of the processing methods for measuring blood flow from the temperature maps;
Figure 4 shows a method for estimating the blood flow rate in the vascular system of a subject using estimated blood flow rates;
Figure 5 illustrates the process of generating a computational fluid dynamics, CFD, simulation;
Figure 6 shows a first validation experiments of the use of CFD simulations for estimating blood flow rate;
Figure 7 shows a 3D model of a realistic blood vessel;
Figure 8 shows the results of a second validation experiment using the 3D model shown in Figure 7;
Figure 9 shows a time-varying blood flow profile;
Figure 10 shows an improved comparison method using pattern detection;
Figure 11 shows a method for estimating the blood flow rate in the vascular system of a subject using outlet resistance values; and
Figure 12 shows an further alternative method for estimating the blood flow rate.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

A system for estimating the blood flow rate in the vascular system of a subject, the system comprising a processor configured to execute memorized instructions to obtain at least two temperature measurements over time of at least two positions in the vascular system and generate a plurality of computational fluid dynamic, CFD, simulations of temperatures in the vascular system using the geometry of the vascular system as boundary conditions, wherein each CFD simulation corresponds to respective CFD input parameters. The processor is further configured to obtain, from each CFD simulation, simulated temperatures over time at positions corresponding to the positions in the vascular system and estimate the blood flow rate in the vascular system including comparing the temperature measurements over time to the simulated temperatures over time corresponding to different CFD input parameters.

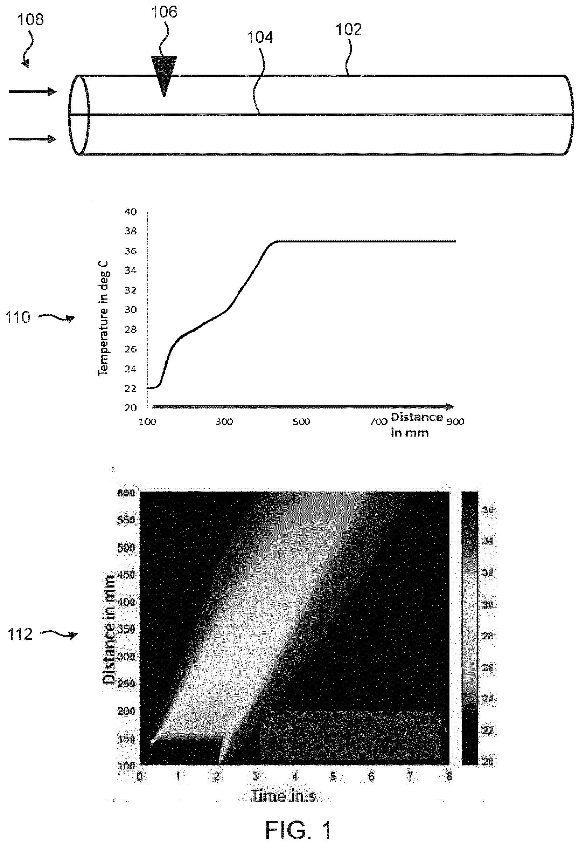

Figure 1 shows an illustration of a fiber optic real shape, FORS, device 104 for measuring the temperature inside a vessel 102 and corresponding simulated temperatures 110 and 112. FORS technology has been very successful for estimating the shape of a catheter 104 and/or guidewire deployed inside the body. It can also be used for temperature measurement along the guidewire centerline. This results in a 2D plot of temperature as a function of time and position. Thus, by injecting a cold bolus at a point 106 in the lumen and at a controlled time, it is possible to track its propagation along the catheter/guidewire 104 centerline, and thus derive the flow velocity by fitting a straight line. The cold bolus could, for example, be a cold saline bolus or a cold contrast bolus. Preferably the cold bolus has relatively low viscosity.

The temperature plots 110 and 112 are illustrations of the envisioned estimation of flow from temperature measurements with FORS technology combined with a cold saline bolus injection at a controlled time. Plot 110 shows the difference in temperatures along the length of the FORS device 104 at a particular time after the injection of the cold bolus at 106. Temperature (in degrees Celsius) is shown along the y axis whilst distance (in mm) along the FORS device 104 is shown in the x axis. Plot 112 shows a temperature map based on the differences in temperature along the length of the FORS device 104 in time after the injection of the cold bolus at 106. The distance (in mm) along the FORS device 104 is shown in the y axis and the time (in seconds) is shown in the x axis. All temperature maps will be shown with distance (in mm) along the y axis and time (in seconds) along the x axis.

During the propagation along the vessel of the cold bolus, the cold bolus mixes with the ambient blood flow 108 which smooths the edges of the temperature map 112 due to thermal diffusion and flow mixing. Moreover, the pulsatile nature of the blood flow, the distorted shape of the vessel 102 and the catheter/guidewire 104 trajectory off from the vessel centerline induce some additional errors on the estimated flow rate. Computation fluid dynamics, CFD, simulations performed with a FORS guidewire inserted in a straight vessel can be used to illustrate those artefacts.

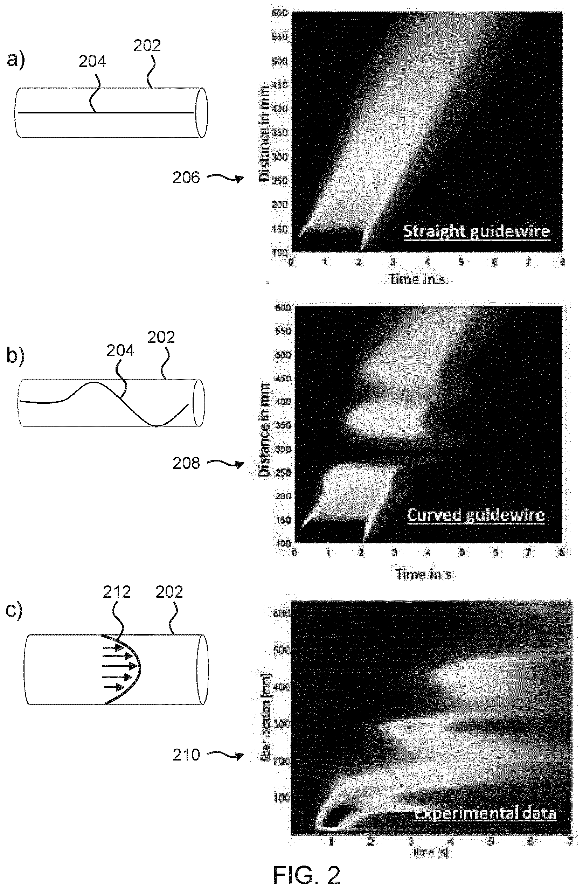

Figure 2 shows simulated temperatures 206 and 208 and an experimental temperature measurement 210. The guidewires 204 in Figures 2 a) and b) have different geometries. In general, the ideal scenario is illustrated in Figure 2 a) which shows a straight guidewire 204 positioned in the center of the vessel 202, with a cold bolus (20°C) injected on its proximal part. The temperature map 206 shows the changes in temperature in the ideal case. The bolus propagates consistently along a diagonal line on the temperature map 206. The diagonal line can be used for the velocity estimation despite the mixing and diffusion smoothing at the bolus edges which brings its temperature closer to the blood temperature (37°C).

However, in a more realistic scenario, the guidewire 204 may be curved. The more realistic scenario is illustrated in Figure 2 b) which shows that the resulting temperature map 208 is significantly affected, in particular the bolus appears to arrive at a later time, is slower and is more blurred as a consequence of diffusion where the guidewire gets close to the wall of the vessel 202. This makes the flow estimation from the temperature map quite challenging, even before the pulsatile nature of the flow and the distorted shape of the vessels is considered.

For comparison, Figure 2 c) shows a temperature map 210 obtained from experimental data. The experimental data is obtained from a guidewire which is curved (relative to the vessel). The issue with temperature map 210 is that the bolus travels at different speeds in the cross-section of the vessel (i.e. slower near the wall and faster in the center). The mixing of the bolus with blood in combination with the flow profile inside the vessel causes the effects seen in the temperature map 210.

For context, a typical Poiseuille flow 212 is illustrated in a vessel 202. As can be seen by the shape of the flow 212, the speed of the flow near the walls of the vessel 202 is slower than the speed in the center. Poiseuille-type flow was used to illustrate the effects on having the catheter/guidewire 204 near the wall. However, other types of flow or other flow profiles could also be used as, in general, most flow profiles will have a slower speed near the walls than in the center of the vessel 202. An annular flow may be assumed to take account of the present of a guidewire in the vessel.

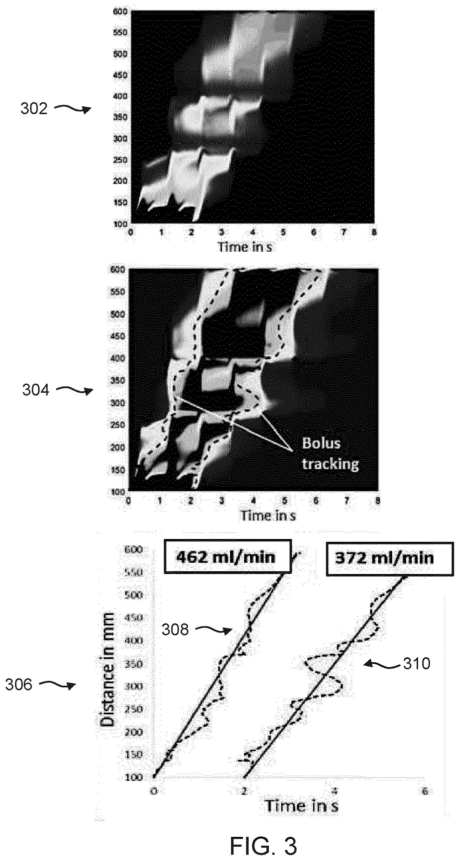

Thus, more advanced processing methods have been developed to tackle the challenge of heat diffusion along the length of the FORS device. Figure 3 illustrates one of the processing methods for measuring blood flow from the temperature maps. Temperature map 302 shows simulated temperatures from a curved guidewire in a straight vessel, wherein the blood flow is a pulsatile flow. Temperature map 304 shows a normalized version of temperature map 302. By normalizing the temperature map 302, the edges of the bolus can be better tracked on both edges. The tracked edges from the normalized temperature map 304 are shown in plot 306. From the tracked edges 308 and 310, it is possible to fit a straight line to estimate the average flow velocity and then the flow rate.

However, these measurements tend to lead to an overestimation of the flow rate on each side of the cold bolus. The tracked edges 308 and 310 lead to measurements of 462 ml/min and 372 ml/min respectively. However, the flow for the simulation was dialed to 300 ml/min. On top of that, the diameter of the blood vessel must be known to convert estimated velocity from the temperature maps into a flow rate value.

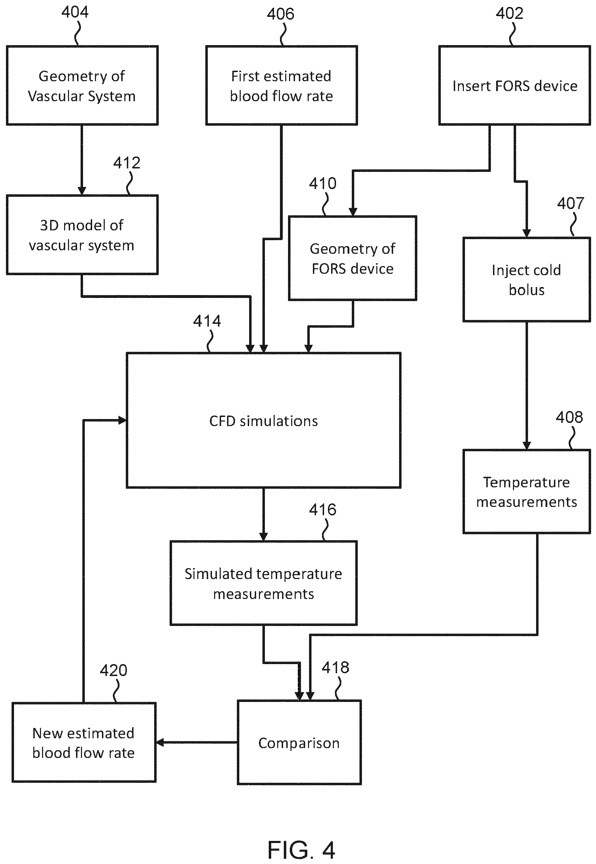

Figure 4 shows a method for estimating the blood flow rate in the vascular system of a subject using estimated blood flow rates as CFD input parameters. The method generally comprises obtaining real temperature measurements (i.e. obtaining temperature maps) as previously described (i.e. after cold bolus injection) and comparing the real temperature measurements with simulated temperatures obtained from a CFD simulation which aims to simulate the flow of blood within a 3D model of the vascular system of the subject.

A FORS device is inserted into the vascular system of the subject in step 402. The FORS device may be inserted in a particular vessel of interest. The geometry of a FORS device inside the vascular system is obtained, in step 410, alongside temperature measurements along the length of the FORS device over time, in step 408. The temperature measurements from the FORS device are obtained after the injection of a cold bolus into the vascular system, in step 407.

A method of injection may be via a sheath or catheter close to the guidewire. However, in some cases the injection site may be relatively far from the FORS device as the bolus will travel through the vessel and, so long as the temperature difference between the bolus and the blood is measurable, the position of the injection site relative to the FORS device is irrelevant. In one example, the cold bolus is injected using a separate device (i.e. not via the catheter/guidewire). Of course, having the precise location of the injection site may increase the accuracy of any measurements using the simulated injection.

CFD simulations of the blood flow and temperatures in the vascular system are generated in step 414. In this case, the CFD simulations are generated iteratively (further explained below). The CFD simulations are generated using the geometry of the vascular system as boundary conditions. The geometry of the vascular system is obtained (e.g. CT images of the vascular system) in step 404 and a 3D model of the vascular system can be generated, in step 412, from the geometry data.

The CFD simulations include a model of the FORS device based on the geometry of the FORS device. Additionally, the CFD simulations include simulation of the injection of the cold bolus into the vascular system (step not shown in Figure 4). Each CFD simulation corresponds to a respective estimated blood flow rate.

Simulated temperatures for each CFD simulation are obtained in step 416 and they are compared to the temperature measurements of the FORS device in step 418.

One aspect to consider for the CFD simulations is the input blood flow rate. The CFD simulations require an input blood flow rate to simulate the blood flow and thus a choice needs to be made as to which so called "estimated blood flow rates" are used in the CFD simulations. In this case, the process iteratively improves an initial guess by comparing the output simulated temperatures with the temperature measurements and generating a new estimated blood flow rate based on the comparison. Thus, for the first CFD simulation, an initial guess (i.e. first estimated blood flow rate) is obtained, in step 406, and new estimated blood flow rates are iteratively obtained based on the previous comparison. The iteration process may be continued until the difference between the temperature measurements and the simulated temperatures is minimal.

The first estimated blood flow rate may be an educated guess of the blood flow. For example, a rough calculation of the blood flow could be obtained from the FORS measurement and used as the first estimated blood flow rate. A population average blood flow rate of the particular vessel of interest could also be used.

In other words, optimization is performed with the CFD simulations using the steady state inlet blood flow rate as a variable parameter and, for example, standard non-linear least square fitting methods (e.g. Levenberg- Marquardt, Gauss-Newton, Gradient methods, Direct search methods etc.) until the difference between the measured and simulated temperature map converges to a minimum. This converged blood flow parameter can then be the provided as a clinical output.

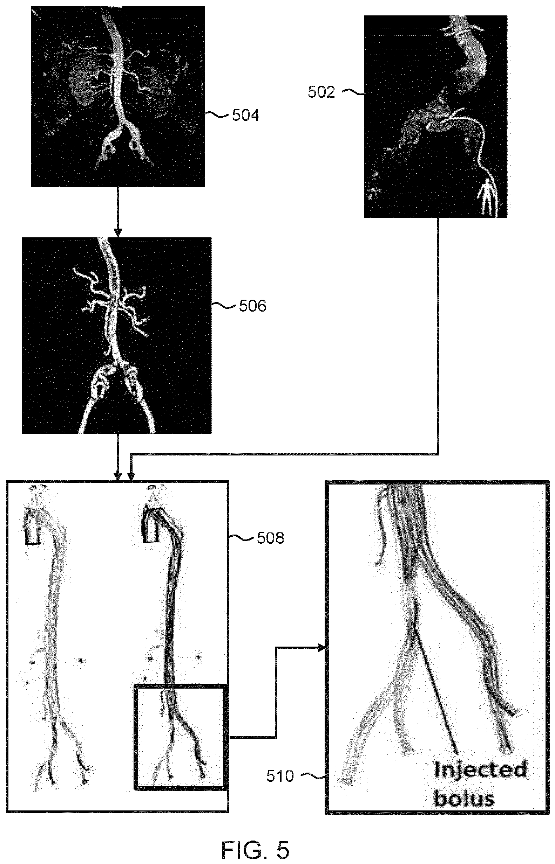

Figure 5 illustrates the process of generating a CFD simulation. The image data 504 is obtained with, for example, computer tomography angiography, CTA, magnetic resonance angiography, MRA, or rotational angiography. Ultrasound may also be used including intravascular ultrasound. From the image data 504, a 3D geometry of the vessel 506 is generated, which is used to describe the vessel anatomy in the CFD simulation 508 and thus act as boundary conditions. Based on the spatial information 502 from the FORS technology, the 3D shape of the guidewire/catheter is determined relative to the vessels and also included in the CFD simulation 508. The injection of the cold bolus (e.g. of saline) is included in the CFD simulation 508 using knowledge of its volume and injection time from the clinical protocol, and optionally also the bolus injection rate. The simulated injection can be seen as a change in temperature in the CFD simulation 508. Image 510 shows a zoomed in version of the CFD simulation 508.

The time varying temperature and flow fields along the guidewire/catheter are simulated with the CFD simulation 508 using an initial guess of the inlet blood flow rate. The simulated temperatures can thus be obtained from the simulations. In general, the CFD simulation and corresponding simulations of the blood flow and temperature are meant to imitate as closely as possible the blood flow in the vascular system of a subject. The simulated temperatures, at different guesses of blood flow rate, can thus be compared to the real temperature measurements and the closest simulated temperatures (i.e. with least differences) can be used to estimate the real blood flow rate based on the (estimated) blood flow rate for which the closest simulated temperatures were taken.

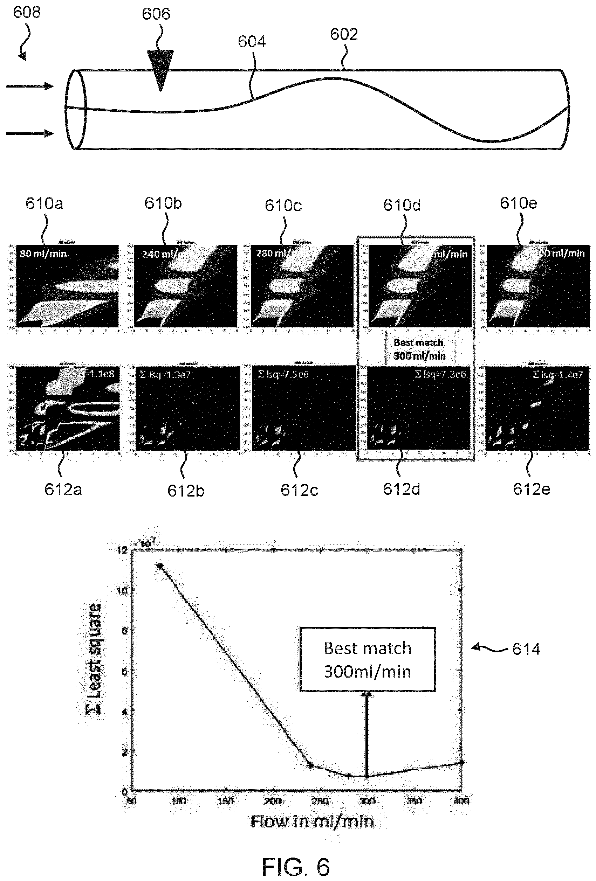

Figure 6 shows a first validation experiment of the use of CFD simulations for estimating blood flow rates. A curved guidewire 604 was inserted in a straight vessel 602 mimicking the femoral artery. A blood flow of 300 ml/min was used as the input blood flow in the vessel 602, shown at 608.

The first validation experiment was first performed with a physiological pulsatile flow in order to generate temperature maps mimicking the experimental data measured with FORS technology. The resulting experimental temperature measurements were similar to temperature map 302 shown in Figure 3.

The optimization method using CFD simulations above was applied to the same respective geometry in a CFD simulation using a steady state flow as a variable parameter. The temperature maps 610a - 610e show the temperature measurements of the simulations at five different estimated blood flow rates.

Temperature map 610a corresponds to a blood flow rate of 80 ml/min, temperature map 610b corresponds to a blood flow rate of 240 ml/min, temperature map 610c corresponds to a blood flow rate of 280 ml/min, temperature map 610d corresponds to a blood flow rate of 300 ml/min and temperature map 610e corresponds to a blood flow rate of 400 ml/min.

The mean squared difference between the experimental data and the temperature maps 610a - 610e are calculated as a cost function. Maps 612a - 612e show the differences between the experimental measurements and the simulation measurements 610a - 610e respectively. The sum of the differences was calculated for each of the maps 612a - 612e in order to determine the closest temperature map.

Map 612a has a sum of 1.1x10⁸, map 612b has a sum of 1.3x10⁷, map 612c has a sum of 7.5x10⁶, map 612d has a sum 7.3x10⁶ and map 612e has a sum of 1.4x10⁷.

The sums of maps 612a - 612e are plotted on graph 614 where the blood flow "guess" is on the x-axis and the least square sum is on the y-axis. The convergence towards the mean experimental flow rate is clearly demonstrated in the optimization plot 614 (i.e. towards 300 ml/min). This validates the proposed mapping procedure of the invention.

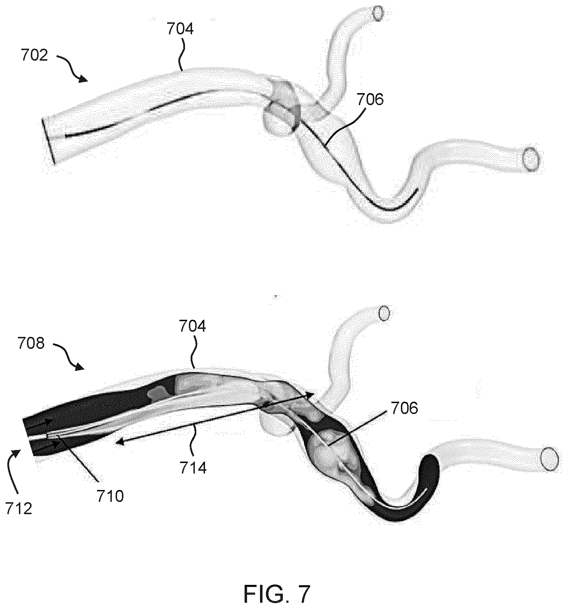

A second validation was also performed with a more realistic vessel geometry. Figure 7 shows a 3D model of a realistic blood vessel 704. A curved guidewire 706 is inserted in the more realistic blood vessel 704 corresponding to the bifurcation from the abdominal aorta into the common iliac arteries. The 3D model 702 was reconstructed from CT images.

CFD simulation snapshot 708 shows the changes in temperature within the vessel 704 due to the injection of a cold bolus at point 710 into the steady state blood flow input 712. The length 714 shows the mixing area where the cold bolus mixes with the warmer blood. Experimental temperature measurements for an identical vessel geometry were obtained at an average blood flow of 746 ml/min.

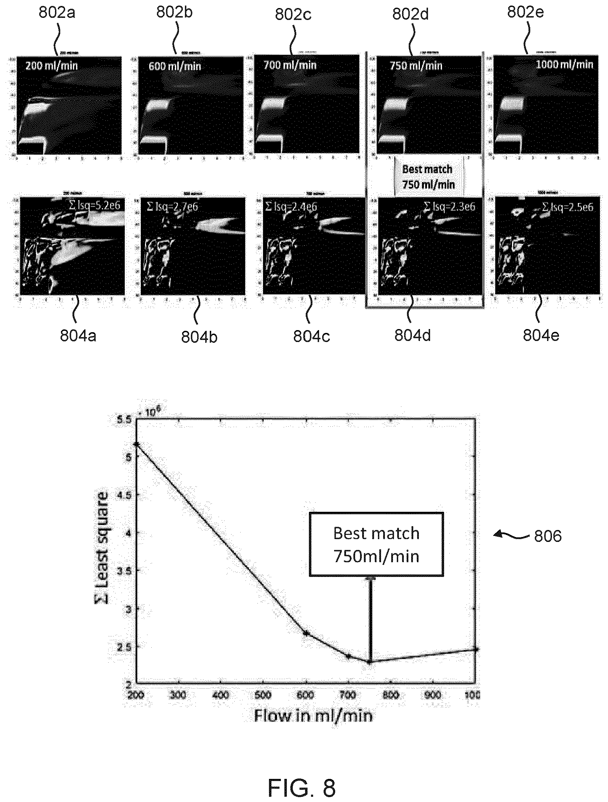

Figure 8 shows the results of a second validation experiment using the 3D mode shown in Figure 7. Temperature maps 802a - 802e show temperature measurements for five different average blood flow values used in five CFD simulations as shown in Figure 7.

Temperature map 802a corresponds to an estimated blood flow rate of 200 ml/min, temperature map 802b corresponds to an estimated blood flow rate of 600 ml/min, temperature map 802c corresponds to an estimated blood flow rate of 700 ml/min, temperature map 802d corresponds to an estimated blood flow rate of 750 ml/min and temperature map 802e corresponds to an estimated blood flow rate of 1000 ml/min.

The maps 804a - 804e show a comparison between the steady state temperature maps 802a - 802e at different flows and the temperature measurements mimicking experimental data measured with FORS technology.

The mean squared difference between the experimental data and the temperature maps 802a - 802e are calculated as a cost function. Maps 804a - 804e show the differences between the experimental measurements and the simulation measurements 802a - 802e respectively. The sum of the differences was calculated for each of the maps 804a - 804e in order to determine the closest temperature map.

Map 804a has a sum of 5.2x10⁶, map 804b has a sum of 2.7x10⁶, map 804c has a sum of 2.4x10⁶, map 804d has a sum 2.3x10⁶ and map 804e has a sum of 2.5x10⁶.

The sums of maps 804a - 804e are plotted on graph 806 where the blood flow "guess" is on the x-axis and the least square sum is on the y-axis. The optimization plot 806 demonstrates convergence toward the mean experimental flow rate (i.e. 746 ml/min) as the best match from the five temperature maps 802a - 802e was temperature map 802d. This further validates the proposed mapping procedure of the invention.

The two validation examples previously discussed compared simulated temperature measurements using steady state blood flow to experimental temperature measurements which used a pulsatile blood flow. However, the CFD simulations may also use a simulated pulsatile blood flow.

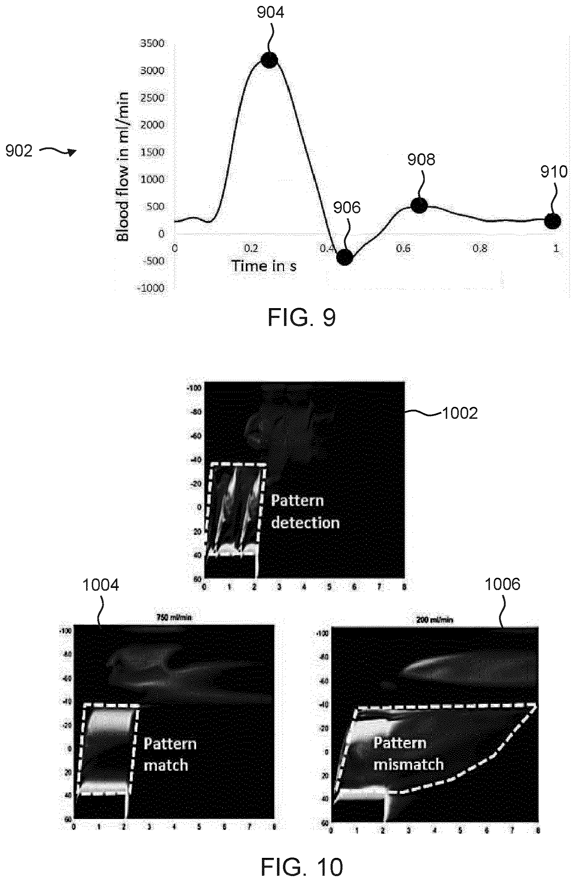

Figure 9 shows a time-varying blood flow profile. Comparing the experimental data and CFD simulation may be further improved by determining the time varying blood flow rate in an iterative manner (e.g. using a generalized PV blood flow profile such as the profile shown in plot 902) instead of using a steady-state average blood flow value.

In this case, the CFD simulation includes a time-varying blood flow, thus further increasing the accuracy of the measurements. The use of time-varying blood flows may be dependent on the complexity and processing requirements to generate the CFD simulation.

Plot 902 is an example of a flow profile of the abdominal aorta. The blood flow rate (ml/min) is shown in the y-axis and the time (s) is shown in the x-axis. This flow rate can be either fully discrete or determined from a generic shape driven by a few key parameters (e.g. pulse period, systole and diastole durations and/or the amplitudes of the systolic peak 904, forward diastolic peak 908, reverse diastolic peak 906 and end diastole 910).

Additionally, the heart rate can be used as an input to make the profile more subject specific. The heart rate can be deduced from an ECG or from the temperature measurements from the FORS catheter/guidewire. For example, the pulse period may be dependent on the heart rate.

Comparing experimental data and CFD simulations to determine the time varying blood flow rate in an iterative manner further comprises using more elaborate optimization algorithms.

The comparison methods previously presented in Figures 6 and 8 rely on the minimization of the mean squared distance between the experimental data and the steady state simulations. This distance is calculated on the full temperature map.

However, more elaborate comparison methods (or optimization algorithms) may be applied. For example, optimization algorithms may focus on more relevant parts of the temperature map. Figure 10 shows an improved comparison method using pattern detection. Temperature map 1002 corresponds to experimental data whilst temperature maps 1004 and 1006 correspond to temperature maps obtained from CFD simulations at two different blood flow rates.

The relevant parts of a temperature map can be determined using pattern detection algorithms as shown on Figure 10. These pattern detection algorithms are based on Artificial Intelligence, Machine learning on trained dataset or deep learning with neural network. The use of these algorithms may improve the robustness, speed and accuracy of the optimization process (e.g. faster comparisons may allow for more iterations).

The pattern detection algorithm used on the temperature maps of Figure 10 shows a pattern match between experimental temperature map 1002 and simulated temperature map 1004. Meanwhile, the pattern detection algorithm shows a mismatch between the patterns of temperature maps 1002 and 1006.

Using pattern detection may improve the accuracy of the comparison method and may additionally help in the comparison between steady state and pulsatile flow.

Using the CFD model, it is possible to extract additional parameters that are clinically relevant such as the outlet resistance(s) and/or pressure drop between the inlet and outlet. Returning to Figure 7, the outlet resistances could be calculated for two downstream outlets at the right of the geometry through which the simulated blood flow leaves the 3D vessel model, if a measured pressure value is obtained. Thus, a subject-specific outlet resistance may be provided as an additional clinical output to the interventionist (e.g. next to the converged flow rate).

The outlet resistance can be defined as the ratio between the (measured) pressure and the flow and used as a subject specific boundary condition in the CFD simulation. Other, more complex, definitions for the outlet resistance may also be used. The pressure can be measured in the same vessel or at another location or even via external sensors. The outlet resistance value brings some valuable insight on the (vessel specific) downstream vessel disease state (e.g. diffuse stenosis, hypertension, microembolization etc.)

The outlet resistance may be calculated by solving the Navier-Stokes equation to obtain the ratio between the pressure and flow and measuring the pressure of the subject.

Additionally, the outlet resistance(s) obtained from the CFD simulations can be iteratively optimized in order to reduce the difference between the measured and simulated temperature maps. The outlet resistances could thus be the CFD input parameters which are optimized instead of the estimated blood flow rates.

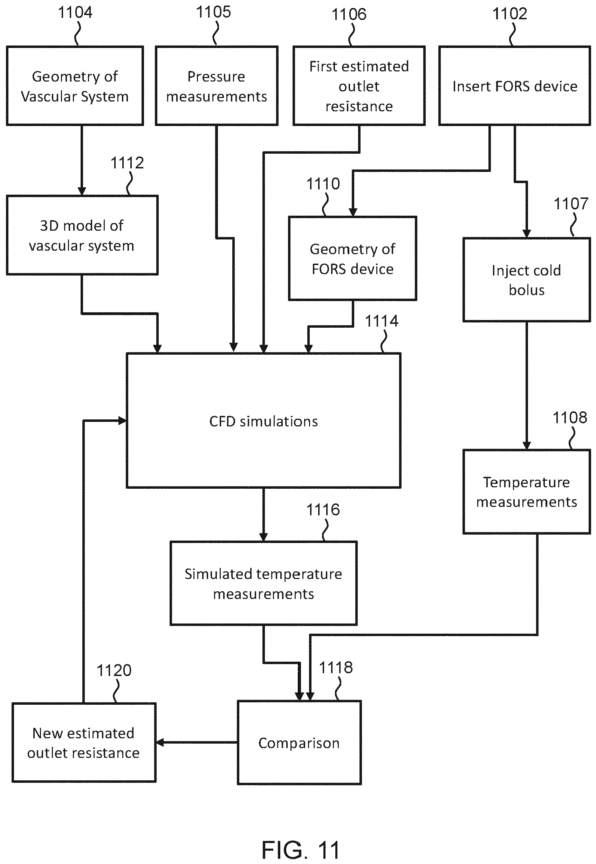

Figure 11 shows a method for estimating the blood flow rate in the vascular system of a subject using outlet resistance values. In addition to the 3D model of the vascular system 1112, pressure measurements 1105 are used as boundary conditions for the CFD simulations 1114 and the downstream outlet resistance(s) could then be optimized. A first estimated outlet resistance value 1106 is used for the first CFD simulation and, based on the comparison 1118 between the simulated temperatures 1116 and the temperature measurements 1108, new estimated outlet resistance values can be obtained and optimized iteratively in the CFD simulations.

As before, this method is based on the insertion of a FORS device 1102 and the injection of a cold bolus 1107. Thus, the geometry 1110 of the FORS device can be obtained and used to inform where to obtain the simulated temperatures 1116. The injection of the cold bolus 1107 is used to inform when to obtain the temperature measurements 1108. The 3D model of the vascular system 1112 is obtained from geometry measurements 1104 of the vascular system.

Comparing the experimental data and CFD simulations in step 1118 to estimate the time varying blood flow rate in an iterative manner can further be combined with a measured time varying pressure to estimate outlet resistance.

When estimating the outlet resistance, the intravascular time varying pressure may be measured which, when used as a boundary condition for the CFD simulations, results in a transient flow profile. A single outlet resistance value can be used for all outlet vessels in the particular geometry of the vascular system or a specific resistance value could be used for each outlet vessel.

The use of time varying outlet resistance values brings some valuable insight on the (vessel specific) downstream vessel disease state (e.g. diffuse stenosis, hypertension, microembolization etc.) and on the physiological process of flow regulation (e.g. following druginduced hyperaemia). Moreover, the CFD simulations can be combined with more advanced boundary conditions. For example, the Windkessel model captures the capacitive effect of the downstream vasculature.

In a further embodiment, comparing the experimental data and CFD simulations to determine the time varying blood flow rate in an iterative manner is further based on successive cold bolus injections injected at a controlled frequency.

Successive cold boluses (e.g. saline or contrast boluses) may be injected at a controlled frequency over a large period of time (i.e. larger the time it takes to inject and measure one bolus). This facilitates the clinical procedure by only setting a saline injection flow rate and frequency, without needing to time accurately the start of the FORS temperature measurement, preventing the risk of missing the bolus. Besides the clinical convenience, having a temperature map with several successive bolus may improve the accuracy of the final temperature map, by smoothing the noise through averaging for example.

The CFD simulation may be displayed on top of the images of the vascular anatomy. Thus, the converged results of the CFD simulation may be displayed to the 3D anatomy to visualize the velocity and pressure field of the blood flow. This may further provide additional clinically relevant information such as the relative blood flow at different vessel branches, pressure gradients across stenosis, shear stresses on the vessel walls etc.

In some cases, only two temperature measurement points along the length of the FORS device are required. Thus, at least two time-varying temperature measurement at different locations are used instead of temperature along the whole length of the catheter/guidewire. The location of those two points need to be known relative to the vessel anatomy. The measurements at these two points can thus be compared to the corresponding points in the CFD simulation.

In an alternative embodiment, 2D images may be used to generate a 2D geometry model of the vascular system and the temperature measurements may be obtained close to the vessel walls. The use of 2D geometry can be treated the same as when using 3D geometry. As the 2D geometry is based on a 2D projection, only non-depth information is available and thus the only reliable location of the catheter/guidewire relative to the vessel cross-section may be the locations that appear close to the wall on the 2D image. Thus, when using 2D geometry, it may be advantageous to only use the temperature measurements and simulated temperatures near the vessel walls.

In summary, a new system is proposed to improve the blood flow estimation from FORS temperature measurement combined with a controlled cold bolus injection. This system relies on a mapping procedure (i.e. comparing) between experimental data and CFD simulations, taking into account the blood vessel geometry, the catheter/guidewire shape and its position relative to the blood vessel.

For example, the mapping procedure can be divided into the following steps:
- Acquisition of vessel image data from an imaging modality (e.g. ultrasound, CT, MRI etc.);
- Reconstruction of the vessel geometry (from the vessel image data);
- Acquisition of the catheter/guidewire shape and of the temperature along its centerline (FORS) relative to a time controlled saline cold bolus injection;
- Generation of a CFD simulation including the FORS catheter/guidewire and the 3D vessel geometry as input data; and
- Simulation of the blood flow and temperature using the CFD simulation.

The bolus flow rate and injection time are known from, for example, the clinical protocol. The inlet blood flow rate is initially unknown and thus the first CFD simulation may start with an initial guess of the blood flow rate. An iterative process on the inlet blood flow can thus take place to minimize the difference between the simulated and measured temperature on the catheter/guidewire centerline.

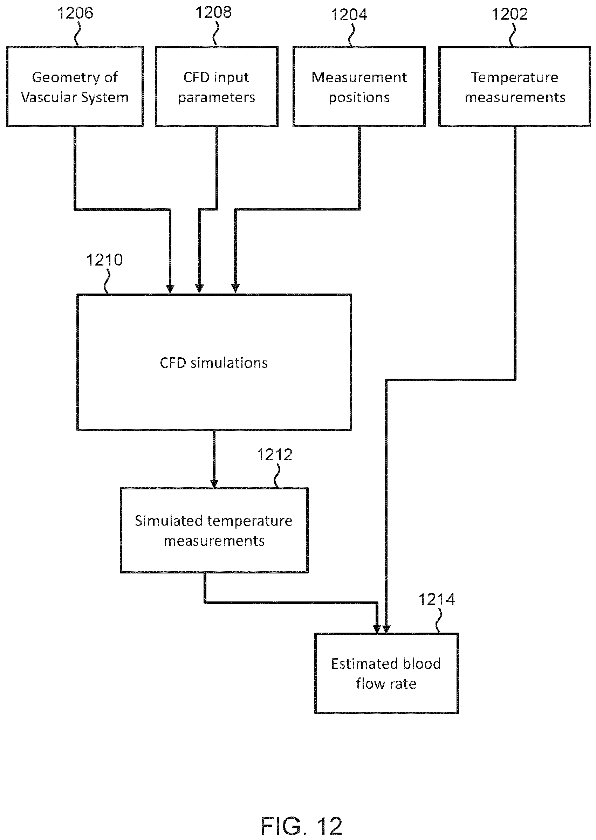

Figure 12 shows an further alternative method for estimating the blood flow rate. Temperature measurements and the corresponding positions are obtained in steps 1202 and 1204 respectively.

At least two temperature measurements are needed from within the vascular system. Two temperature sensors, at two different positions in a vessel, could be used to obtain the two measurements. Alternatively, one of the temperature measurements could be derived from the injection site and when the injection took place (i.e. start and finish time of the injection). Of course, the more temperature measurements which are obtained, the more accurate the blood flow estimation will be. Thus, preferably a FORS device (or similar device) is used.

The temperature measurements are over time and thus appropriate time resolution in the measurements is important. Particularly low temporal resolutions, relative to the time taken for the temperature in the vessel to return to its original temperature, may result in erroneous estimations of the blood flow.

Localization of the temperature measurements (i.e. obtaining the positions) may be done externally (e.g. X-ray, ultrasound, CT etc.) prior to, during or after the temperature measurements. For example, the sensors used to obtain the temperature measurements have radiopaque markers or other detectable markers (e.g. electromagnetic coils). Alternatively, position sensors may be used or the positions may be input manually by a clinician (e.g. located on a CT or ultrasound scan). For a catheter/guidewire comprising two or more markers, the positions of the markers may be interpolated to obtain a continuous geometry of the catheter/guidewire.

When using a FORS device for the temperature measurements, the geometry of the FORS device relative to the vascular system may be augmented (or even replaced) using further localization techniques (e.g. imaging the FORS device). Obtaining the geometry of the FORS device relative to the vascular system is, in essence, equivalent to the localization of the positions of the FORS device.

The geometry of the vascular system is obtained in step 1206. The geometry could be obtained, for example, by an external imaging system (e.g. CT, MRI, OCT, ultrasound etc.) or by an internal imaging system (e.g. intravascular ultrasound, IVUS, catheter). The geometry is used as a boundary condition for the CFD simulations.

CFD input parameters are chosen/estimated for the CFD simulations in step 1208. As previously mentioned, the CFD input parameters may be iteratively optimized through each CFD simulation. The CFD input parameters may comprise estimated blood flow measurements or outlet resistance values with pressure measurements as further boundary conditions.

The CFD simulations can then be generated in step 1210 and simulated temperatures can be obtained in step 1212 from the CFD simulations. The simulated temperatures are obtained at the location corresponding to the positions such that the temperature measurements and the simulated temperatures both correspond to the same location relative to the vascular system and can therefore be compared. Based on the comparisons, a blood flow rate can be obtained in step 1214. Outlet resistance values could also be obtained from the CFD simulations.

The example above is based on the injection of a cold bolus, and modeling of the cooling as the bolus mixes with the blood flow. However the same concept may be applied to a warm bolus, i.e. above body temperature. The injection of the bolus may be a continuous injection instead of successive discrete bolus injections

There may be a heating or cooling device for warming or cooling the blood itself to create a warm or cold bolus.

The skilled person would be readily capable of developing a processor for carrying out any herein described method. Thus, each step of a flow chart may represent a different action performed by a processor, and may be performed by a respective module of the processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

**1.** A system for estimating the blood flow rate in the vascular system of a subject, the system comprising a processor configured to execute memorized instructions to:
receive (1202) at least two temperature measurements over time of at least two positions in the vascular system;
generate (1210) a plurality of computational fluid dynamic, CFD, simulations of temperatures in the vascular system using the geometry of the vascular system as boundary conditions, wherein each CFD simulation corresponds to respective CFD input parameters which are associated with blood flow features;
obtain (1212), from each CFD simulation, simulated temperatures over time at positions corresponding to the positions in the vascular system; and
estimate (1214) the blood flow rate in the vascular system by comparing the temperature measurements over time to the simulated temperatures over time associated with different CFD input parameters.

**2.** The system of claim 1, wherein the processor is configured to iteratively generate the CFD simulations with CFD input parameters which are generated based on a comparison of the simulated temperatures of the previous CFD simulations with the temperature measurements, wherein the first CFD simulation of the iterative generation of CFD simulations is generated using first estimated CFD input parameters.

**3.** The system of any one of claims 1 or 2, wherein the CFD input parameters represent periodic pulses of flow rate profiles of blood from the heart.

**4.** The system of any one of claims 1 to 3, wherein the CFD input parameters represent estimated blood flow rates.

**5.** The system of claim 4, wherein the estimated blood flow rates are based on measurements of the heart rate of the subject.

**6.** The system of any one of claims 1 to 5, wherein the CFD input parameters include a measurement of the pressure in the vascular system used as a boundary condition for the CFD simulations and the processor is adapted to estimate outlet resistance values.

**7.** The system of claim 6, wherein the pressure measurements comprise pressure measurements over time.

**8.** The system of claims 6 or 7, wherein the processor is further configured to estimate outlet resistance values in the vascular system by comparing the temperature measurements over time to the simulated temperatures over time corresponding to different CFD input parameters.

**9.** The system of any one of claims 1 to 8, wherein comparing the temperature measurements to simulated temperatures comprises inputting the temperature measurements and simulated temperatures into a pattern detection algorithm and comparing the detected patterns.

**10.** The system of any one of claims 1 to 9, further comprising a tracking device provided with temperature sensing functionality to obtain said temperature measurements, and arranged to measure the positions for said at least two temperature measurements.

**11.** The system of any one of claims 1 to 9, further comprising a device provided with temperature sensing functionality to obtain said temperature measurements, and arranged with shape sensing to determine the shape and positions of at least sections of the device including the positions for said at least two temperature measurements, wherein the determined shape is optionally used by the processor to determine the geometry of the vascular system or to identify an anatomical region of the vasculature system.

**11.** The system of any one of claims 1 to 10, further comprising an imaging system for obtaining the geometry of the vascular system.

**12.** The system of any one of claims 1 to 11, wherein the CFD simulations further include a simulation of an injection of a temperature altering bolus.

**13.** The system of claim 12, wherein the processor is further configured to analyze quality parameters of the temperature measurements and output a temperature measurement feedback to a user interface.

**14.** A method for estimating the blood flow rate in the vascular system of a subject, the method comprising:
obtaining (1202) at least two temperature measurements over time of at least two positions in the vascular system;
generating (1210) a plurality of computational fluid dynamic, CFD, simulations of blood flow and temperatures in the vascular system using the geometry of the vascular system as boundary conditions, wherein each CFD simulation corresponds to respective CFD input parameters which are associated with blood flow features;
obtaining (1212), from each CFD simulation, simulated temperatures overtime at positions corresponding to the positions in the vascular system; and
estimating (1214) the blood flow rate in the vascular system by comparing the temperature measurements over time to the simulated temperatures over time corresponding to different CFD input parameters.

**15.** A computer program product comprising computer program code which, when executed on a computing device having a processing system, cause the processing system to perform all of the steps of the method according to any of claims 10 to 14.
